# EUROPEAN PATENT APPLICATION

(11) **EP 3 904 493 A1**
(43) Date of publication of application: **03.11.2021**
(21) Application number: 20172453.1
(22) Date of filing: 30.04.2020
(51) Int. Cl.: C12M 1/00, C12M 1/08

(54) **PHOTOBIOREACTOR**

(71) Applicant: O&N B.V., 8701 DV Bolsward (NL)
(72) Inventor: VAN VELZEN, Dick, 8701 DV Bolsward (NL); MULDER, Gerardus Rudi Maria, 8701 DV Bolsward (NL); OOSTERBAAN, Wiebe IJsbrand Leo, 8701 DV Bolsward (NL); HULSHOFF, Hendrik Jan, 8701 DV Bolsward (NL); STEL, Jarno, 8701 DV Bolsward (NL); OELEN, Jan Johannes, 8701 DV Bolsward (NL); JAGER, Filips Gustaaf Hendrik, 8701 DV Bolsward (NL); MAESSEN, Theodorus Antonius, 6096 CH Grathem (NL); VAN DRUNEN, Rudi, 8701 DV Bolsward (NL); KOEK, Charles Adrianus Gerardus, 8651 EB Ijlst (NL)
(74) Representative: van Dam, Vincent

(57) **Abstract**

The present invention relates to a bioreactor and a method for growing organisms capable of photosynthesis in an aqueous liquid. The bioreactor and method of the invention are suitable for all kinds of organisms which are capable of photosynthesis and growing in an aqueous medium.

## Description

The present invention relates to a bioreactor and a method for growing organisms capable of photosynthesis in an aqueous liquid. The invention also relates to a method for increasing production of algae derived lipids and/or carotenoids in said bioreactor.

Organisms capable of photosynthesis convert light energy into chemical energy by converting carbon dioxide and water into the end-products oxygen and carbohydrates. This process is energized by light.

Algae form an example of such organisms, and are well-known as efficient producers of biomass. During photosynthesis, algae utilize carbon dioxide and light in the presence of water to produce oxygen and carbohydrate biomass. The algae produce lipids, vegetable oils and proteins (and other forms of phytobiomass) which can be used for various purposes, for example as a source for human or animal nutrition or biofuel.

Algae grow best under controlled conditions. For instance, algae are sensitive to temperature and light conditions and in particular also fluctuations thereof. Algal yield may be improved by controlling growth parameters such as temperature, CO₂ levels, light and nutrient concentration.

Various methods have been used to grow algae but it appears difficult to grow algae efficiently on a commercial scale.

For instance, US patent 8,569,050 discloses a bioreactor for algae comprising an outer containment vessel and a flow tube placed in the containment vessel. The outer containment vessel is provided with LEDs in acrylic spheres along the length of the interior wall of the vessel. Flow of the algae suspension is generated by a bubbler situated in the flow tube which causes the algae to travel to the top of the flow tube where the stream of algae are pushed out of the flow tube into the outer containment vessel to stream downward along the interior wall of the outer containment vessel and exterior of the flow tube where the algae are exposed to LED light from the acrylic spheres facing from the interior wall of the outer containment vessel. Once the algae suspension reaches the bottom of the bioreactor it is drawn into the flow tube with the cycle repeated.

The inventors have found that the present bioreactors, such as the one described in US patent 8,569,050, have their limitations with regard to efficiency.

It is further noted that the present applicant has filed an earlier, yet unpublished European patent application no. 18 204 113.7. This application describes a bioreactor for growing organisms capable of photosynthesis in an aqueous liquid, comprising a tank comprising: an outer chamber having a bottom portion and a top portion and an inner chamber having a bottom portion and a top portion, wherein said inner chamber is configured within said outer chamber, wherein the bottom portion and the top portion of the inner chamber comprise one or more openings to allow fluid connection between the inner and outer chamber, and one or more spargers located in the outer chamber on the bottom portion of the outer chamber and at a lateral distance from the bottom end of the inner chamber, wherein the outer chamber comprises a plurality of LED bars distributed above said one or more spargers, each LED bar extending from the outer wall of the outer chamber in the direction of the inner chamber. Algal flow in this system is caused by a mechanism different from that described in US patent 8,569,050. Although efficiency is highly improved with the bioreactor described in European patent application no. 18 204 113.7, the inventors considered that there is room for even further improvement.

### Summary of the invention

In a first aspect the invention relates to a bioreactor for growing organisms capable of photosynthesis in an aqueous liquid, comprising a tank comprising an outer chamber having a bottom portion and a top portion and an outer wall, and at least one inner chamber having a bottom portion and a top portion, wherein said at least one inner chamber is configured within said outer chamber, wherein the bottom portion and the top portion of the at least one inner chamber comprise one or more openings to allow fluid connection between said at least one inner chamber and said outer chamber, and at least one sparger located in the outer chamber in the bottom portion of the outer chamber and at a lateral distance from the bottom end of said at least one the inner chamber, wherein the outer chamber comprises a plurality of LED bars, wherein the individual LED bars extend from the top portion of the outer chamber in the direction of the bottom portion of the outer chamber.

When in operation, the bioreactor is filled with an aqueous liquid with said organisms in it. In this case said tank contains an aqueous liquid with said organisms in it, wherein at the bottom and the top portion of the inner chamber(s) one or more of said openings of the inner chamber are fully submerged in said aqueous liquid to form fluid connections between the inner chamber(s) and outer chamber, and wherein the plurality of LED bars in the outer chamber are submerged in said aqueous liquid.

In a second aspect the invention relates to a method for growing organisms capable of photosynthesis in an aqueous liquid in the bioreactor in operation according to the first aspect, said method comprising providing a circulation of said growing organisms between said outer and said at least one inner chamber by bubbling gas from said at least one sparger, so that said growing organisms flow from bottom to top in the outer chamber and from top to bottom in said at least one inner chamber, thereby exposing the organisms in the flowing aqueous liquid to said LED bars in said outer chamber.

In a third aspect the invention relates to a method for increasing production of algae derived lipids and/or carotenoids, comprising providing in the bioreactor of the first aspect a circulation of algae between said outer chamber and said at least one inner chamber by bubbling gas from said at least one sparger, so that said algae flow from bottom to top in the outer chamber and from top to bottom in said at least one inner chamber; and applying stress conditions to the algae.

The use of LED bars extending from the top portion of the outer chamber in the direction of the bottom portion of the outer chamber, in particular in an essentially vertical manner, ensures light exposure of all microorganisms flowing upwards through the outer chamber, because the flowing microorganisms will in principle be exposed to light over the full length of the LED bars while ascending to the top portion of the outer chamber.

The LED bars extend from the top portion of the outer chamber in the direction of the bottom portion of the outer chamber, preferably in a downward fashion. This allows LEDs to be removed and replaced from the outside of the tank, for instance via the top lid of the tank without the need for disassembling the bioreactor. This allows replacement and maintenance of LEDs without the necessity of emptying the tank or any other interruption of the culture process. This allows almost continuous operation of the bioreactor of the invention.

The LEDs in the bars have the advantage that they offer the versatility to accommodate light requirement variations amongst various microorganisms, such as in particular algal species. LEDs are also safer and more efficient than traditional lighting sources.

The use of LED light sources for algal growth however also has some disadvantages which are associated to the fact that, apart from light, LEDs produce also heat albeit in a much reduced quantity compared to conventional light sources. In fact, up to half of the electricity supplied to a LED is converted to heat rather than light (∼ 40-50 % heat: 50-60 % light). This heat production may have detrimental effects on the efficiency of the bioreactor used for growth and propagation of the algae. When LEDs are used in a bioreactor for algae, it is in general preferred that LEDs are encapsulated by a housing of light transmitting material. Preferred in this respect is a polycarbonate housing, in particular in light of limitations to the materials allowed to be in contact with food masses in production. This housing is then submerged in the algal growth medium. Because of the heat produced by the LEDs the light transmitting material of the housing will also be heated. If the temperature of the housing surface becomes too high, this may cause caking of algae onto the surface of the housing, obscuring the LEDs from the growing algae and thus lowering the efficiency of the reactor.

The inventors have surprisingly found that the provision of one or more bubble blowing spargers, which in particular blow bubbles at a low rate, located in the outer chamber in the bottom portion of the outer chamber and at a lateral distance from the bottom end of the at the least one inner chamber, causes less caking of microorganisms such as algal cells, to the LED bars and in particular to their housing.

At the same time the gas bubbles drive the flow cycle of the medium. In this way circulatory flow of medium and microorganisms is realized in a gentle manner which avoids undue stress for the microorganisms, which in its turn is beneficial for sustaining uninterrupted microorganism growth and biomass accumulation.

In view of the above, the provision of both the sparger(s) and the LED bars in the outer chamber results in the advantages of:
1) optimal LED light exposure of growing microorganisms;
2) easy replacement and maintenance of LEDs without the necessity of emptying the tank allowing continuous operation;
3) prevention of caking of microorganisms on the LED bars;
4) reduced stress for microorganisms.
   Moreover, the specific configuration of the LED bars extending downwards from the top portion the outer chamber in the direction of the bottom portion of the outer chamber also allows:
5) almost unlimited expansion in the diameter or width of the bioreactors, because it allows the use of multiple inner chambers in a single outer chamber.

These advantages contribute to high efficiency and reduce in operating costs of production of the bioreactor and method of the invention.

### Short description of the figures

Fig. 1 shows a side view cross section of a bioreactor of an embodiment of the bioreactor of the invention.
Fig. 2 shows a schematic representation of a top view cross section of the bottom portion along line A of the bioreactor of Fig.1.
Fig. 3A shows a perspective view of the construction inside the outer chamber of another embodiment of the bioreactor according to the invention containing multiple vertical inner chambers. Fig. 3 B shows a top view of the construction of Fig. 3A.
Fig. 4 shows in detail how the spargers in the setup of Fig. 3 are configured. Fig. 4A shows a perspective view of the sparger assembly. Fig. 4B shows areas covered with bubbles produced by the sparger loops as seen from above. Fig. 4C shows details of a part of the sparger assembly of Fig. 4A as seen from above.
Fig. 5 (perspective view) shows a supporting frame in which the inner chamber cylinders can be mounted.

### Detailed description of the invention

In principle the bioreactor and method of the invention are suitable for all kinds of organisms which are capable of photosynthesis and growing in an aqueous medium. It is preferred that the organisms are phytoplankton or algae, such as microalgae, because these organisms produce lipids, vegetable oils and proteins which can be used for various purposes such as a source for human or animal nutrition or biofuel.

The bioreactor according to the invention comprises a tank comprising an outer chamber having a bottom and a top portion and an outer wall, and at least one inner chamber having a bottom portion and a top portion, wherein said at least one inner chamber is configured within said outer chamber, wherein said one or more of said openings allow fluid connection between said at least one inner chamber and said outer chamber via the bottom and the top portion of the inner chamber. Because the inner chamber is placed in the outer chamber, the wall of an inner chamber preferably separates the outer and inner chamber from each other.

The principle of the bioreactor of the present invention is fully applicable to a bioreactor with a single inner chamber. However, it is preferred that the bioreactor comprises multiple of said inner chambers configured within an outer chamber. This allows almost unlimited volumes of algae to be grown in a single outer chamber as defined by its outer lining or in a single bioreactor. The use of multiple inner chambers in a single outer chamber is made possible by the downward, in particular vertical, arrangement of the LED bars.

In an embodiment an inner chamber has open top and bottom ends to provide said one or more of said openings in the bottom and top portion of the inner chamber to allow fluid connections (at both ends) between the inner and outer chamber. When fluid connection between the inner and outer chamber is realized by an open top end and an open bottom end of the inner chamber this causes an optimal entry and exit in and out the inner chamber. This allows for a reduction of mechanical stress for the microorganisms, which on its turn is advantageous for growth and propagation.

In this respect the at least one inner chamber preferably has an open top end to provide a single opening in the top portion of the inner chamber. Alternatively the top end of the inner chamber has multiple openings to provide a fluid connection at the top end.

The bottom end of the inner chamber may also have a single opening at one end of the inner chamber, but is it preferred that it has multiple openings in its wall, because this allows orientation of the organisms streaming into the outer chamber.

In a preferred embodiment the fluid connection between the inner and outer chamber at least at the bottom end of the inner chamber is realized by the provision of openings oriented such that the algae exit the inner chamber in the horizontal plane. In a vertical inner chamber that means that the openings are directed orthogonally with respect to the longitudinal axis of the inner chamber. This results in broader expansion of the algae in the outer chamber in the circumferential direction outwards of the inner chamber, which on its turn is advantageous for uniform dispersion of the algae in the outer chamber, and thus exposure to light.

In a suitable embodiment the outer chamber is defined by a first vertical cylinder and the at least one inner chamber is defined by a second vertical cylinder, wherein the at least one inner chamber is mounted into said first vertical cylinder as a further "second cylinder".

In the bioreactor of the invention one or more spargers are located in the outer chamber in the bottom portion of the outer chamber and at a lateral distance from the bottom end of the at least one inner chamber. The sparger (s) can be suitably mounted on the bottom (floor) of the outer chamber. The spargers are configured to bubble gas into the liquid growth medium containing the microorganisms in the outer chamber. The bubbling frequency is preferably chosen as low as possible and it is preferred that bubble size is limited. This is to further reduce stress exposure. Said gas may be air, nitrogen or carbon dioxide or any mixture thereof. The spargers may be in any form capable of blowing gas bubbles into the growth medium.

In a preferred embodiment the gas released by the spargers in the bottom portion of the outer chamber is air. This is preferably filtered air, such as HEPA filtered air.

The bubbling gas causes a local decrease in the density of the growth medium, causing upward flow through the outer chamber. As soon as the bubbles have reached the upper surface the gas contained in the bubbles will be released causing the density of the medium to increase. As a result the denser medium will enter the at least one inner chamber which is not provided at its bottom with a bubble generating means and the denser medium will flow downwards through the at least one inner chamber. The bottom of the at least one inner chamber is in fluid contact with the medium in the outer chamber so that the down flowing medium that has reached the bottom of the at least one inner chamber will be caused to enter the outer chamber which is provided with said bubble generating means so that the medium is forced to flow up again. This way a circular flow is created. When in operation the inner chamber is preferably dimensioned to fully fit into the outer chamber and be fully submerged in the growth medium.

Nutrients may also be added. It is preferred that the nutrients at least comprise CO₂. These may also be added via the spargers. However, nutrients are preferably introduced into the top portion of the at least one inner chamber. In this respect the bioreactor is preferably provided with a nutrient supply means configured to introduce nutrients, such as CO₂, into the flowing medium from the top portion of the at least one inner chamber. De-coupling the air supply and nutrient supply this way has the advantage that the nutrients will be fully dissolved and uniformly dispersed in the inner chamber. When the organisms enter the outer chamber, photosynthesis commences again with all nutrients being uniformly dispersed in the medium. This ensures uniform growing conditions for all organisms, which is advantageous because it allows optimal control of the growth and propagation conditions, which on its turn is beneficial for efficiency.

Preferably CO₂ is injected into the inner chamber from the top portion. For this purpose, preferably a source of CO₂ is injected in liquid form. An equilibrium between CO2 and carbonate wil set during the medium's downward flow through the inner chamber in which generally dark conditions occur and thus hardly or no photosynthesis takes place. At the moment the medium reaches the bottom portion of the inner chamber the equilibrium will be set. Upon entry into the outer chamber the organisms will be exposed to light, triggering photosythesis, and thus increasing their demand for CO₂. Injection of CO2 into the inner chamber from the top portion thus ensures that the necessary amount of CO₂ can be controlled and is fully available to the organisms at entry into the outer chamber.

The vertical position of the sparger(s) may overlap with the vertical position of the inner chamber or be below the bottom end of the inner chamber. In accordance, the sparger(s) may be located at a position below the opening(s) in the bottom portion of the inner chamber, at the same height as the opening(s) or above the opening(s).

It is also preferred that the spargers are located below said LED bars. This is advantageous because the inventors have found that the injection of gas below said LED bars leads to a minimum of caking of microorganisms to the LED bars.

Said one or more spargers are also located at a lateral distance from the bottom end of the inner chamber. This allows unhindered circulation of the liquid growth medium. The lateral distance is chosen such that bubbles are injected in the outer chamber and cannot accidentally enter into the inner chamber.

In one embodiment multiple spargers may be distributed in the bottom portion of the inner chamber. Preferably the spargers are distributed evenly to provide an even distribution of gas bubbles to the liquid growth medium, providing all microorganisms with an equal driving force.

In a preferred embodiment the bioreactor comprises a sparger extending in the outer chamber in a loop at a lateral distance from the bottom end of the inner chamber and around the circumference of the inner chamber. In this embodiment a single sparger per inner chamber suffices if the bioreactor contains a single inner chamber. Such a loop may be easily removed and replaced for maintenance. The loop is preferably removable from above. For this purpose, it is preferred that the vertical position of the loop is above the exit opening (s) of the inner chamber (s). In case of a single inner chamber, preferably the loop is mounted onto the bottom of the outer chamber and runs around the inner chamber at a lateral distance from its bottom end at a position approximately in the centre between the wall of the inner chamber and the wall of the outer chamber. Also this provides an even distribution of gas bubbles to the liquid growth medium, providing all microorganisms with an equal driving force and metabolic gas in case carbon dioxide is (co-) dosed in the outer chamber.

Further in this respect it is noted that in an embodiment wherein multiple inner chambers are provided in a single outer chamber, a loop at a lateral distance from the bottom end of the inner chamber and around the circumference of the inner chamber each loop will effect in a bubble path in the direction of the top portion of the outer chamber. Such a bubble path will also extend to a certain extent in the circumferential direction both inwards and outwards of the loop. However, this may not always be not sufficient to fill the full trans-sectional area of the outer chamber with equal quantities of bubbles, and as such can result in so-called dead zones between adjacent inner chambers or between inner chambers and the wall of the outer chamber, in which dead zones no bubbles rise if no further provisions would be taken. This has a potential disadvantageous effect on circulation efficiency because algae may end up in the dead zones and float or descend to the bottom as the unwanted result of the creation of downward streams of bubble-free, "dense" water outside of the inner chambers, or sediment or stick to the wall of the outer chamber. To prevent this, the spargers are preferably arranged such that the full outer chamber, at least where the liquid medium flows, is fully filled with equal or essentially equal concentrations of ascending bubbles. This can for instance be realised by the provision of linear spargers extending from the centre of the bioreactor and through these dead zones and/or by providing at least one sparger distributed along the inner circumference of the outer chamber. The latter also prevents sticking of algae to the outer chamber walls. In a preferred embodiment therefore the bioreactor further comprises at least one sparger distributed along the inner circumference of the outer chamber.

As mentioned above, the gas bubbles cause a flow of said microorganisms upwards through the outer chamber / cylinder. During their way up, the microorganisms pass and travel along the plurality of LED bars. When the bioreactor is operational the bars extend in the liquid growth medium from a top portion of the outer chamber to the bottom of the outer chamber. Although it is possible that the LED bars are somewhat angularly directed with respect to the longitudinal axis of the bioreactor, it is highly preferred that the LED bars, in particular each LED bar extending in the medium, extends vertically from the top portion of the outer chamber in the direction of the bottom portion of the inner chamber. This allows equal light distribution over all of the ascending culture medium and including organisms and advantageous use of available space because the inner chamber(s) will in general also be arranged vertically.

Preferably the LEDs are arranged on the LED bar in a triangular arrangement of the bar which enables that the LED arrays or rows of LEDs of the three surfaces of the bar are spaced apart 120°. This allows 360° of light emission. This can be realised by using a LED holder with a first end and a second end and three elongated outer walls extending between the first and second end, wherein each wall is laterally connected to the other two walls by a water impermeable connection, and wherein the walls are oriented with respect to each other to define a space of substantially equilateral triangular cross section.

To allow optimal light exposure of the organism in the bioreactor, preferably the individual LED bars extend from the top portion of the outer chamber over the majority of the length between the top portion and the bottom portion of the outer chamber. More preferably the individual LED bars extend from the top portion of the outer chamber over the majority of the length between the top portion and the bottom portion of the outer chamber up to close above the at least one sparger, for instance essentially up to the at least one sparger.

As mentioned above, the use of LED lamps in the form of LED bars extending from the top portion of the outer chamber in the direction of bottom portion of the outer chamber allows LEDs to be removably placed without the need to replace the housing or having to disassemble the bioreactor.

In a preferred embodiment therefore said LED bars comprise LED arrays comprising multiple LEDs in tubes of light transmitting material, preferably polycarbonate tubes, which LED arrays are removable from the top portion of the outer chamber. This may be realized by arranging the LED bars in tubes of light transmitting material extending from the upper cover or lid of the bioreactor. For this purpose, the tubes of light transmitting material may each have a receiving portion at the lid of the outer chamber which allows inserting and removing LED arrays in any of said tubes.

In a further preferred embodiment tubes of light transmitting material extend from the top portion of the outer chamber to the bottom portion and are attached to the bottom of the outer chamber via a LED support assembly. This allows the LED bars to be kept in place under all circumstances.

It is preferred that the LED bars comprise a tubular or generally tubular housing of light transmitting material surrounding the LED arrays in spaced relationship with the LEDs on the arrays, wherein said LED arrays extend over most of the length of said tubular housing. This light transmitting material may be of any light transmitting material such as glass or plastic, preferably polycarbonate.

Because the bars are fully submerged in the liquid growth medium when the bioreactor is operational and are light transmitting in all directions lateral from its longitudinal axis, exposure of the microorganisms to LED light is enhanced compared to other LED systems used in the art.

For the same reason, the LED arrays are preferably configured such that LED light is emitted in all directions around the longitudinal axis of the bar.

Because the bioreactor of the invention allows control of any growth parameter, such as the light parameter, it is perfectly suitable to manipulate conditions which result in a change of metabolism of the organisms. Therefore, the bioreactor can also be used to increase production of value added products from said microorganisms. Such products comprise in particular lipids, such as poly-unsaturated fatty acids (PUFAs), such as omega 3 fatty acids. In this respect the invention also relates to a method for increasing production of algae derived lipids and/ or carotenoids, comprising providing in the bioreactor of the invention a circulation of algae between the outer and the at least one inner chamber by bubbling gas from said at least one sparger, so that said algae flow from bottom to top in the outer chamber and from top to bottom in the inner chamber(s), and applying stress conditions to the algae.

The application of stress conditions without inhibitory effects of the above mentioned bubbles on the algal cell metabolism may suitably involve the manipulation of growth parameters, such as temperature, light, and nutrient mixture composition.

These stress conditions can lead to the accumulation of lipids in the algae. This makes it possible to commercially produce poly-unsaturated fatty acids (PUFAs), such as omega 3 fatty acids from algae.

After stressing the algae, the algae can be harvested and processed to extract and optionally purify the lipids contained therein.

### Detailed description of the drawings

The invention will now be further elucidated in the attached drawings. The following explanation is meant to illustrate and explain the invention and not to limit the claims.

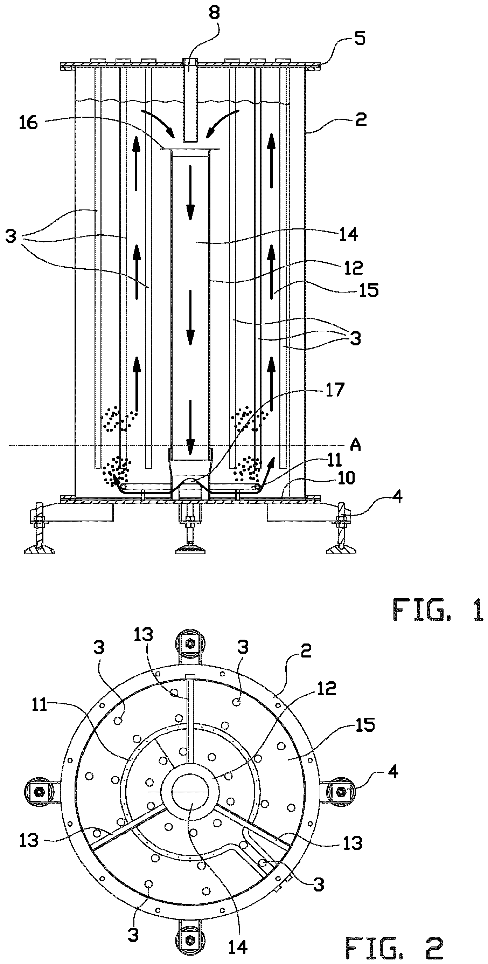

Fig. 1 and 2 show an exemplary embodiment of a bioreactor 1 in accordance with the invention. Fig. 1 shows a side view cross section of a bioreactor wherein the reactor tank is filled with growth medium. Fig. 2 shows a top view cross section along line A of the bioreactor of Fig. 1. The bioreactor of Figs. 1 and 2 allows performing a preferred embodiment of the method according to the invention wherein algae are grown by creating a circulation of algae along the LED bars submerged in the growth medium of the algae in the reactor. The bioreactor of Figs. 1 and 2 comprises a cylindrical tank 2 standing on four feet 4. The tank 2 is closed with a lid 5 onto which means for opening and closing the tank are provided. Multiple LED bars 3 extend vertically from the lid 5 into the tank 2 in the direction of the tank. On the lid of this bioreactor further a pipe for degassing (not shown), a sealable pipe 8 for water supply and nutrients, and a water level detector (not shown) are provided. At the bottom of the tank a bottom plate 10 is provided. Alternative to a flat bottom plate also a curved bottom plate, can be used (not shown) for instance of glass fiber reinforced polyester to provide extra strength. Via this bottom plate gas can be provided into the tank via pipe 11.

On the top side an air inlet (not shown) may optionally be provided (optionally with a HEPA filter) for purposes of preventing vacuum in case of opening air valves (not shown) in the lower portion of the reactor such as in the lower portion of the inner cylinder (not shown).

Inside tank 2 an inner cylinder 12 is placed supported by supporting members 13. The inner cylinder 12 defines an inner chamber 14. On the other hand the remainder of the cylindrical tank 2 now defines an outer chamber 15 surrounding inner chamber 14. In the embodiment shown in Fig 1 and 2 therefore the outer chamber 15 is defined by a first vertical cylinder, which is formed by the tank 2, and the inner chamber 12 is defined by a second, namely inner vertical cylinder, wherein the second vertical cylinder is mounted into said first vertical cylinder. When in operation the tank is filled with an aqueous growth medium containing algae. The level of growth medium should be such that the full inner chamber is submerged in the growth medium. To ensure that the level of growth medium is maintained at acceptable or optimal levels the tank 2 is provided with sealable pipe 8 for water supply, and a water level detector (not shown). Flow of the growth medium is effected by the provision of a pipe 11 with pores which extends on the bottom of the tank in a circumferential fashion as a loop around the inner cylinder at a suitable distance from it to provide a means for introducing gas such as air into the outer chamber 15, i.e. it functions as a sparger. This causes a local decrease in the density of the growth medium in the outer chamber causing an upward flow (see arrows for the direction of flow) . During the way up the algae in the growth medium are exposed to the light emitted by LED bars 3 which are provided with a tubular housing and which extend in the growth medium vertically from the lid 5 of the tank 2 to the bottom part of the outer chamber 15 in the tank 2 and which are evenly distributed throughout the outer chamber 15. As shown in Fig.2 the LED bars 3 extend from the top portion of the outer chamber 15 between the top portion and the bottom portion of the outer chamber 15, up to close above the sparger 11. As soon as the bubbles have reached the upper surface the gas contained in the bubbles will be released on the surface of the growth medium, causing the density of the growth medium to increase locally. As a result the denser medium will enter the inner chamber 14 via opening 16 which is provided at the top end to provide a single opening in the top portion of the inner chamber 14. Because of its higher density the medium flows downwards. The now heavier growth medium leaves the inner chamber via bottom outlets 17 and enters the outer chamber 15. There the medium is expanded again by gas bubbles released from the pores in pipe 11, causing the lightened medium to flow up again in the outer chamber 15, thus effecting a circulation.

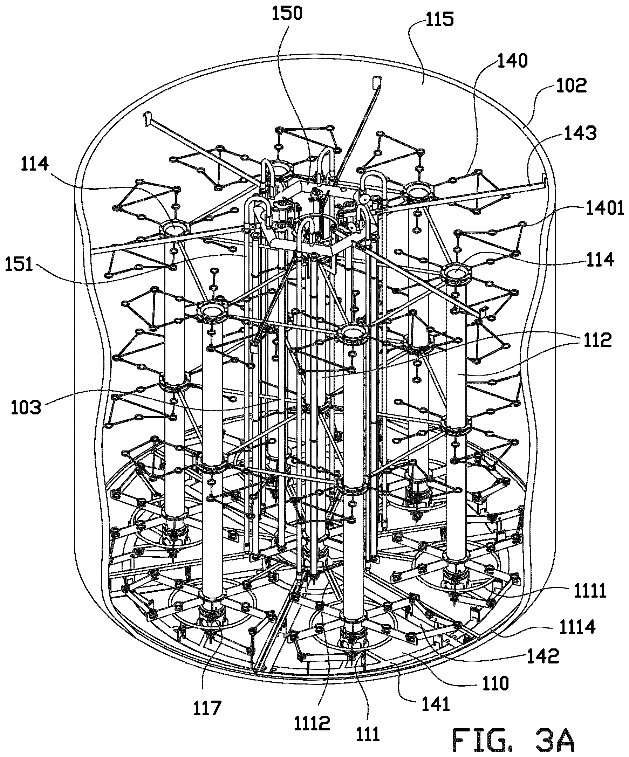

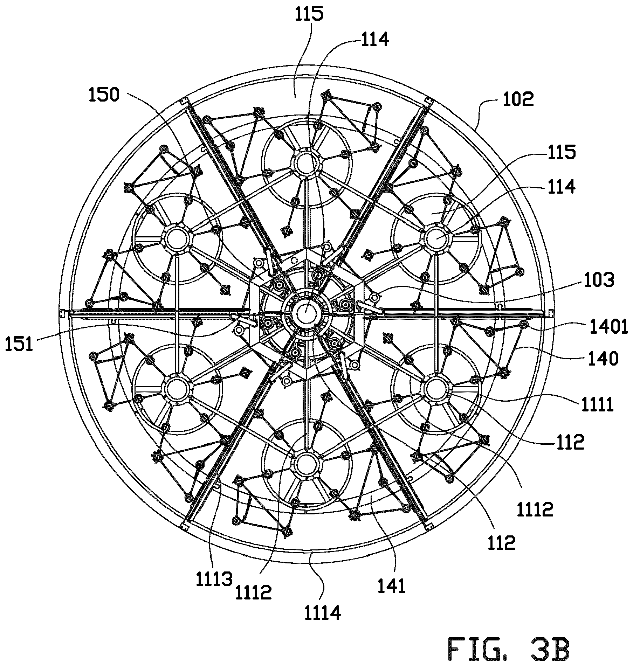

In a preferred embodiment a bioreactor according to the invention comprises multiple of said inner chambers configured within said outer chamber. An example of such an embodiment will now be explained in reference to Figs. 3 to 5. Fig. 3A shows a perspective view of such a bioreactor wherein the outer chamber wall 102 is shown only partially to allow visualisation of the inside of the bioreactor. Fig 3B shows a top view of this bioreactor.

The outer chamber wall 102 forms a cylindrical tank which is closed with a lid onto which means for opening and closing the tank are provided. For purposes of visualisation this lid is not shown for this embodiment. Multiple LED bars 103 extend vertically from the lid in the direction of bottom of the tank. The LED bars 103 are provided with a tubular housing and are supported by LED bar support members 140. In Fig. 3A only part of the LED bars 103 are shown for purposes of oversight. Therefore the holes 1401 in the LED support members 140 are not all filled, but it is clear that a high number of LED bars can be vertically arranged in this bioreactor. Just like the embodiment of Figs. 1 and 2 also here the lid of the bioreactor may comprise a pipe for degassing (not shown), a sealable pipe for water supply, and a water level detector are provided (not shown). At the bottom of the tank a bottom plate 110 is provided, or alternatively, a curved bottom as described for Fig.1 and 2.

Inside the tank multiple inner chambers 114 are provided by vertical cylinders 112 supported by supporting members 141, 142, 143. The inner cylinders 112 thus define the inner chambers 114. On the other hand the cylindrical tank defined by outer wall 102 surrounds all inner cylinders 112 and defines an outer chamber 115.

When in operation the tank is filled with an aqueous growth medium containing algae. The level of growth medium should be such that the inner chambers 114 are fully submerged in the growth medium. To ensure that the level of growth medium is maintained at acceptable or optimal levels the bioreactor may be provided with a sealable pipe for water supply, and a water level detector (not shown). Flow of the growth medium is effected by the provision of a sparger assembly 111. The sparger assembly 111 has spargers with pores 1115 and extends inter alia on the bottom of the tank in a circumferential fashion as a loop 1111 around the inner cylinders 112 at a suitable distance from it to provide a means for introducing gas such as air into the outer chamber 115, i.e. it functions as a sparger. In this embodiment the vertical position of sparger assembly 111 is above the bottom outlets 117 of the inner chambers. This allows removing the sparger system without having to fully disassemble the bioreactor. This causes a local decrease in the density of the growth medium causing an upward flow (see arrows for the direction of flow) . During the way up the algae in the growth medium are exposed to the light emitted by LED bars 103 which are provided with a tubular housing and which extend in the growth medium vertically from the lid of the tank to the bottom part of the outer chamber 115 . The LED bars are preferably evenly distributed throughout the outer chamber 115 to provide uniform light exposure to the microorganisms. As shown in Fig.3A the LED bars 103 extend from the top portion of the outer chamber 115 between the top portion and the bottom portion of the outer chamber 115, up to close above the sparger assembly 111. As soon as the bubbles have reached the upper surface the gas contained in the bubbles will be released on the surface of the growth medium, causing the density of the growth medium to increase locally. As a result the denser medium will enter the inner chamber 114 via openings provided at the top end. Because of its higher density the medium flows downwards. The growth medium leaves the inner chamber via bottom outlets 117 and enters the outer chamber 115. There the medium is expanded again by gas bubbles released from the pores 1115 in sparger assembly 111, causing the medium to flow up again in the outer chamber 115, thus effecting a circulation.

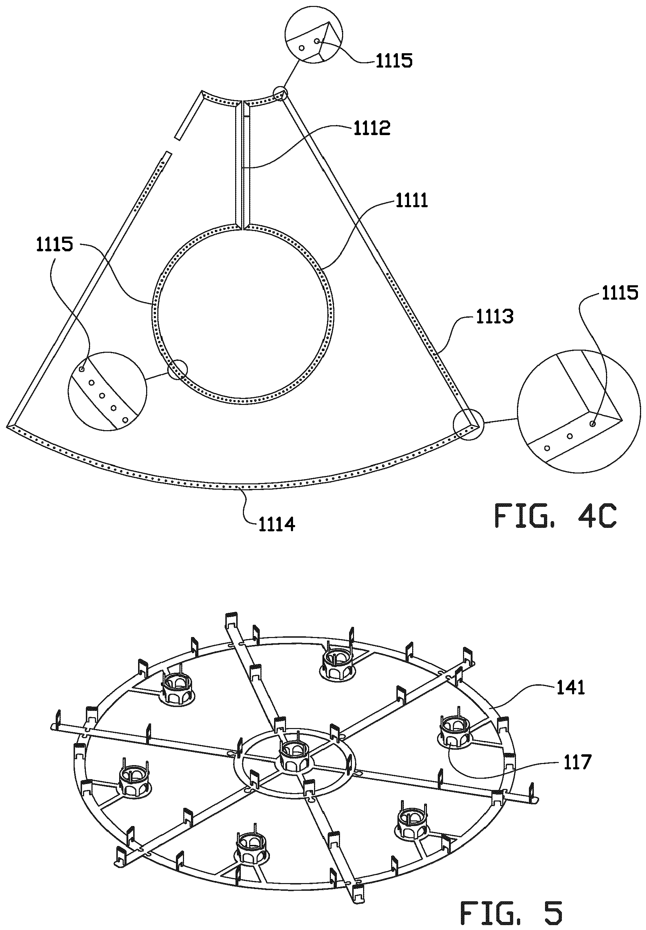

As shown in detail in Fig.5 the bottom outlets 117 of the inner chamber 114 in this embodiment are integrated in support member 141. The inner cylinders 112 can therefore be seated onto bottom outlets 117 of support member 141.

Gas for the sparger assembly 111 is supplied from above via gas inlet manifold 150 and gas supply pipes 151 which are connected to the sparger assembly 111 at the bottom of the tank. Gas inlet manifold 150 is connected to an outside gas source (not shown). It is preferred to provide gas from above because in case of failure or damage of the sparger assembly 111 this prevents water from intruding through the whole gas supply system with accompanied and undesired emptying of the reactor.

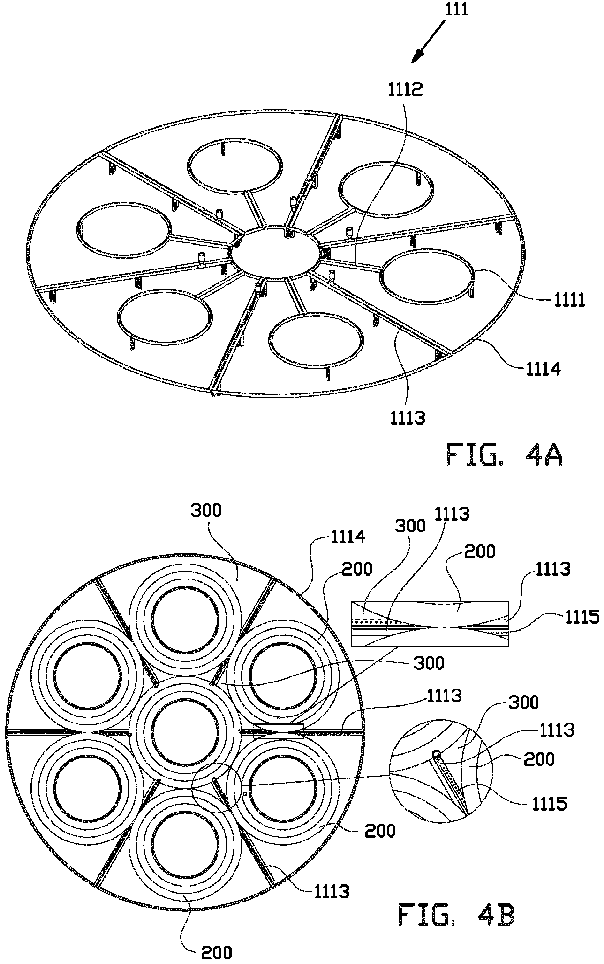

Further reference is made to the sparger assembly 111 as shown in detail in Fig. 4A and C, wherein Fig.4C shows a part of the sparger assembly 111. In particular when loops 1111 around the inner cylinders 112 are provided at a suitable distance from it to provide a means for introducing gas such as air into the outer chamber 115, each loop will effectively create a bubble path in the direction of the top portion of the outer chamber. As seen from above in Fig. 4B this bubble path 200 extends also to a certain extent in the circumferential direction inwards and outwards of the loop. However, this may not be sufficient to cover the full area of the outer chamber, and may result in so-called dead zones 300 where no bubbles rise if no further provisions would be taken. This has a potential disadvantageous effect on circulation efficiency because algae may end up in the dead zones 300 and float or descend to the bottom 110 or stick to the side wall 102 of the outer chamber. To prevent this the sparger assembly 111 is also provided with spargers 1113 extending from the centre of the bioreactor and through these dead zones 300. Also spargers 1114 extending along the inner circumference of the outer chamber wall 102 are provided in this embodiment. In this way, all algae are kept in circulation and sedimentation of algae on the bottom 110 or outer chamber wall 102 is prevented.

## Claims

1. A bioreactor for growing organisms capable of photosynthesis in an aqueous liquid, comprising a tank comprising:
an outer chamber having a bottom portion and a top portion and an outer wall, and at least one inner chamber having a bottom portion and a top portion, wherein said at least one inner chamber is configured within said outer chamber, wherein the bottom portion and the top portion of the at least one inner chamber comprise one or more openings to allow fluid connection between said at least one inner chamber and said outer chamber, and
at least one sparger located in the outer chamber in the bottom portion of the outer chamber and at a lateral distance from the bottom end of said at least one the inner chamber,
wherein the outer chamber comprises a plurality of LED bars, the individual LED bars extending from the top portion of the outer chamber in the direction of the bottom portion of the outer chamber.

2. The bioreactor according to claim 1, which comprises multiple of said inner chambers configured within said outer chamber.

3. The bioreactor according to claim 1 or 2, wherein the fluid connection between the at least one inner chamber and said outer chamber at the bottom end of said at least one inner chamber is realized by the provision of openings oriented such that the algae exit the inner chamber in the horizontal plane.

4. The bioreactor according to any of the previous claims, wherein the outer chamber is defined by a first vertical cylinder and said at least one inner chamber is defined by a second vertical cylinder, wherein each second vertical cylinder is mounted into said first vertical cylinder.

5. The bioreactor according to any of the previous claims, wherein the individual LED bars extend from the top portion of the outer chamber over the majority of the length between the top portion and the bottom portion of the outer chamber, preferably up to close above the at least one sparger.

6. The bioreactor according to any of the previous claims, wherein said at least one sparger extends in the outer chamber in a loop at a lateral distance from the bottom end of the at least one inner chamber and around the circumference of the at least one inner chamber.

7. The bioreactor according to claim 6, further comprising at least one sparger distributed along the inner circumference of the outer chamber.

8. The bioreactor according to any of the previous claims, wherein said LED bars comprise LEDs in tubes of light transmitting material, which LEDs are removable from the top portion of the outer chamber.

9. The bioreactor according to any of the previous claims, wherein the individual LED bars extend vertically from the top portion of the outer chamber in the direction of the bottom portion of the inner chamber.

10. Bioreactor according to any of the previous claims, wherein the organisms capable of photosynthesis are phytoplankton or algae.

11. The bioreactor according to any of the previous claims, wherein said tank contains an aqueous liquid with said organisms in it, wherein at the bottom portion and the top portion of said at least one inner chamber one or more of said openings in said at least one inner chamber are fully submerged in said aqueous liquid to form fluid connections between said at least one inner chamber and the outer chamber, and wherein the LED bars are submerged in said aqueous liquid.

12. The bioreactor according to any of the previous claims, wherein the bioreactor is provided with a CO₂ supply means configured to introduce CO₂ into the inner chamber from the top portion of the at least one inner chamber.

13. A method for growing organisms capable of photosynthesis in an aqueous liquid in the bioreactor according to any of the previous claims, said method comprising providing a circulation of said growing organisms between said outer and said at least one inner chamber by bubbling gas from said at least one sparger, so that said growing organisms flow from bottom to top in the outer chamber and from top to bottom in said at least one inner chamber, thereby exposing the organisms in the flowing aqueous liquid to said LED bars in said outer chamber.

14. The method according to claim 13, wherein said gas bubbled by said at least one spargers is air and wherein CO₂ is introduced into the top portion of the at least one inner chamber.

15. A method for increasing production of algae derived lipids and/or carotenoids, comprising providing in the bioreactor of any of the claims 1 to 12 a circulation of algae between said outer chamber and said at least one inner chamber by bubbling gas from said at least one sparger, so that said algae flow from bottom to top in the outer chamber and from top to bottom in said at least one inner chamber; and
applying stress conditions to the algae,
preferably wherein the application of stress conditions involves the manipulation of growth parameters, such as temperature, light, and nutrients; and/or wherein the lipids comprise poly-unsaturated fatty acids (PUFAs), such as omega 3 fatty acids.
